# FASCICULE DE BREVET EUROPEEN

(11) **EP 1 910 531 B1**
(45) Date de publication et mention de la délivrance du brevet: **21.04.2010**
(21) Numéro de dépôt: 06778825.7
(22) Date de dépôt: 07.07.2006
(51) Int. Cl.: C12N 9/16, C12P 7/18, C12P 3/00, A23K 1/165

(54) **EFFET SYNERGIQUE DE L'ASSOCIATION DE PHYTASES SUR L'HYDROLYSE DE L'ACIDE PHYTIQUE**
SYNERGISTISCHER EFFEKT DER KOMBINATION VON PHYTASEN AUF DIE HYDROLYSE VON PHYTINSÄURE
SYNERGISTIC EFFECT OF THE COMBINATION OF PHYTASES ON HYDROLYSIS OF PHYTIC ACID

(30) Priorité: 08.07.2005 FR 0507335
(43) Date de publication de la demande: 16.04.2008
(73) Titulaire: Adisseo France S.A.S., 92160 Antony (FR)
(72) Inventeur: BOZE, Hélène, F-34070 Montpellier (FR); MOULIN, Guy, F-34980 Montferrier-sur-Lez (FR)
(74) Mandataire: Jouannic, Nathalie
(86) Numéro de dépôt international: PCT/FR2006/001652
(87) Numéro de publication internationale: WO 2007/006952

(56) Documents cités:
- EP-A- 0 619 369
- WO-A-00/71728
- WO-A-98/30681
- WO-A-02/098442
- FUJITA JIN ET AL: "Critical importance of phytase for yeast growth and alcohol fermentation in Japanese sake brewing" BIOTECHNOLOGY LETTERS, vol. 23, no. 11, 1 juin 2001 (2001-06-01), pages 867-871, XP002369734 ISSN: 0141-5492
- NASI M ET AL: "COMPARISON OF ASPERGILLUS NIGER PHYTASE AND TRICHODERMA REESEI PHYTASE AND ACID PHOSPHATASE ON PHYTATE PHOSPHORUS AVAILABILITY IN PIGS FED ON MAIZE-SOYBEAN MEAL OR BARLEY-SOYBEAN MEAL DIETS" ARCHIV FUER TIERERNAEHRUNG - ARCHIVES OF ANIMAL NUTRITION, AKADEMIE VERLAG, BERLIN, DE, vol. 52, no. 1, 1999, pages 15-27, XP008045959 ISSN: 0003-942X
- ZYTA K: "THE ROLE OF ACID PHOSPHATASE ACTIVITY DURING ENZYMIC DEPHOSPHORYLATION OF PHYTATES BY ASPERGILLUS NIGER PHYTASE" WORLD JOURNAL OF MICROBIOLOGY AND BIOTECHNOLOGY, RAPID COMMUNICATIONS OF OXFORD, OXFORD, GB, vol. 9, no. 1, 1993, pages 117-119, XP000614876 ISSN: 0959-3993
- WYSS MARKUS ET AL: "Biochemical characterization of fungal phytases (myo-inositol hexakisphosphate phosphohydrolases): Catalytic properties" APPLIED AND ENVIRONMENTAL MICROBIOLOGY, vol. 65, no. 2, février 1999 (1999-02), pages 367-373, XP002369735 ISSN: 0099-2240
- DVORAKOVA J: "PHYTASE: SOURCES, PREPARATION AND EXPLOITATION" FOLIA MICROBIOLOGICA, PRAQUE, CZ, vol. 43, no. 4, 1998, pages 323-338, XP001080605 ISSN: 0015-5632
- VOHRA A ET AL: "PHYTASES: MICROBIAL SOURCES, PRODUCTION, PURIFICATION, AND POTENTIAL BIOTECHNOLOGICAL APPLICATIONS" CRC CRITICAL REVIEWS IN BIOTECHNOLOGY, CRC PRESS, BOCA RATON, FL, US, vol. 23, no. 1, 2003, pages 29-60, XP008060235 ISSN: 0738-8551

## Description

L'invention concerne l'effet synergique de l'association de phytases sur l'hydrolyse de l'acide phytique.

Les sels de l'acide phytique (myo-inositol hexakis phosphate) ou phytates (myo-inositol hexakis dihydrogène phosphate) sont la forme majeure de stockage du phosphore dans les céréales, les plantes légumineuses et oléagineuses. Ils constituent ainsi la source principale de phosphore dans les aliments pour animaux à base de plantes, composants principaux des régimes des animaux monogastriques (volailles et porcs). Cependant, la biodisponibilité de ce phosphore dans les aliments est limitée pour ces animaux. En effet, ils ne possèdent pas les enzymes intestinales dégradant les phytates en quantité suffisante pour permettre l'hydrolyse des phytates et fournir ainsi les quantités de phosphate inorganique qui leur sont nécessaires. En outre, l'acide phytique est un facteur antinutritionnel, qui forme des complexes avec les protéines et les ions (Fe³⁺, Ca²⁺, Zn²⁺, Mg²⁺) et diminue donc la disponibilité de ces éléments.

Les rations alimentaires des volailles et des porcs doivent donc être supplémentées en phosphate inorganique alors que le phosphore des phytates est excrété et contribue à l'eutrophisation des eaux de surface dans les zones d'élevage intensif d'animaux monogastriques.

Les phytases (myo-inositol hexakis phosphate 3- et 6-phosphohydrolases EC 3.1.3.8 et 3.1.3.26) font partie de la famille des histidines phosphatases acides. Les phytases sont des hexakisphosphohydrolases qui hydrolysent les liaisons phospho-esters présentes dans l'acide phytique ou le phytate. Ainsi, elles catalysent l'hydrolyse du *myo*-inositol hexakisphosphate (acide phytique, InsP₆) en monophosphates inorganiques et en *myo*-inositols phosphate de degré de phosphorylation inférieur (InsP₅ à InsP₁) et en *myo*-inositol libre dans certains cas.

Il existe deux sous classes de phytases, différenciées uniquement par la position du premier phosphate hydrolysé. Les 3-phytases (EC 3.1.3.8) hydrolysent le phosphate en position 3 et les 6-phytases (EC 3.1.3.26) hydrolysent le phosphate en position 6.

Ces enzymes utilisées comme additif en alimentation animale, permettent d'une part d'augmenter la disponibilité du phosphore phytique, d'autre part d'améliorer la digestibilité des aliments. De plus, la libération de phosphate phytique diminue considérablement les coûts dus à la supplémentation en phosphate, ainsi que la pollution provoquée par un excès de phosphates excrétés.

Les phytases sont produites par une grande variété d'organismes : plantes, animaux, et surtout microorganismes. Ces phytases possèdent des caractéristiques biochimiques très différentes en particulier leur activité en fonction du pH et leur stabilité à la température. De plus, ces enzymes présentes des différences au niveau: (a) de leur efficacité à hydrolyser la totalité des phosphates, (b) de leur stéréospécificité, (c) de leur affinité vis-à-vis des inositols phosphates.

Parmi les microorganismes producteurs de phytases on citera notamment : les champignons des genres *Aspergillus, Penicillium, Mucor* et *Rhizopus,* les bactéries : *Pseudomonas* sp., *Klebsiella* sp., *Escherichia coli, Enterobacter* sp., *Bacillus subtilis* et levures : *Saccharomyces cerevisiae, Candida tropicalis, Torulopsis candida, Debaryomyces castellii, Debaryomyces occidentalis (synonyme Schwanniomyces castellii) , Kluyveromyces fragilis.*

De nombreuses phytases de microorganismes ont déjà été étudiées et utilisées dans diverses applications agro-industrielles. L'efficacité de ces enzymes dans l'aliment et au cours de la digestion chez l'animal dépend de leur capacité à maintenir leur potentiel quelles que soient les différentes conditions rencontrées au cours des étapes de préparation de l'aliment ainsi que dans le tractus digestif. La préparation des aliments nécessite des enzymes thermorésistantes alors que les valeurs de pH varient par exemple de 5.02, 2.75, 6.28, 6.63, et 5.98 lors du passage dans les divers compartiments jabot, estomac, duodénum, jéjunum, iléon des volailles.

La plupart des phytases décrites à ce jour n'hydrolysent que partiellement l'acide phytique et certaines avec des cinétiques très lentes. Ainsi, de nombreuses phytases n'hydrolysent que 5 liaisons phosphate de l'acide phytique soit 83% du phosphore potentiel.

A ce jour, parmi les levures, seule la phytase de la levure *Schwanniomyces occidentalis* a été décrite comme capable d'hydrolyser toutes les liaisons phosphate du phytate (EP 0 699 762 et Segueilha L., Lambrechts C., Boze H., Moulin G., Galzy P., (1992) Purification and properties of a phytase from Schwanniomyces castelliii, J. Ferm. Bioeng., 74, 7-11).

En conditions expérimentales, on n'obtient cependant pas une hydrolyse complète du phytate ou de l'acide phytique.

Pour obtenir une hydrolyse plus efficace de l'acide phytique, il a ainsi été proposé de combiner différentes phytases.

WO98/30681 décrit la combinaison de phytases ayant différentes stéréospécificités et notamment la combinaison de 3-phytase et de 6-phytase. Ce document décrit en particulier la combinaison d'une 6-phytase de *Peniophora lycii* et d'une 3-phytase d'*Aspergillus niger* (phytase Novo™).

US 6,183,740 décrit également la combinaison de phytases de différentes stéréospécificités ainsi que la combinaison avec d'autres acides phosphatases.

Une hydrolyse complète de l'acide phytique en phosphate et en inositol n'est cependant pas observée.

Le problème que se propose de résoudre la présente invention consiste à améliorer la vitesse et l'efficacité d'hydrolyse de l'acide phytique afin d'obtenir une hydrolyse complète de l'acide phytique dans diverses applications agro-industrielles.

Ce problème est résolu par les compositions et les procédés de la présente invention dans lesquels on associe au moins deux phytases spécifiques. De façon avantageuse, les compositions de la présente invention permettent une hydrolyse complète de tous les groupements phosphate de l'acide phytique grâce à l'effet synergique observé. Avantageusement, les compositions de la présente invention sont actives dans un plage de pH large adaptée aux applications agro-industrielles des phytases et notamment aux applications dans le domaine de l'alimentation animale. Un autre avantage de la présente invention est que les phytases utilisées sont thermostables.

### Description des séquences

SEQ ID No. 1: Phytase de *Schwanniomyces castellii.*
SEQ ID No. 2: Phytase de *Debaryomyces castellii.*
SEQ ID No. 3: Phytase d'*Aspergillus niger.*
SEQ ID No. 4: Phytase de *Penicillium funiculosum.*
SEQ ID No. 5: Phytase de *Peniophora lycii.*
SEQ ID No. 6: Séquence codant pour la phytase de blé.
SEQ ID No. 7 : Phytase de blé (*Triticum aestivum*).

### Description de l'invention

L'invention a pour objet une composition associant au moins deux phytases pour l'hydrolyse de l'acide phytique (myo-inositol 1,2,3,4,5,6-hexakis phosphate) comprenant :
- une première phytase présentant au moins 80% d'identité avec la phytase de la SEQ ID No. 1 ou présentant au moins 80 % d'identité avec la phytase de la SEQ ID No.2 ;
- une deuxième phytase présentant au moins 80 % d'identité avec la phytase de la SEQ ID No. 3.

De préférence, la première phytase est une 3-phytase catalysant l'hydrolyse des six liaisons phosphate de l'acide phytique.

Préférentiellement, la première phytase a une température optimum comprise entre 55°C et 80°C et un pH optimum compris entre pH 3.5 et pH 5.

Dans un mode de réalisation préféré, la première phytase est une phytase de la levure *Schwanniomyces castellii* ou une phytase de la levure *Debaryomyces castellii_{.}*

Dans un autre mode de réalisation préféré, la première phytase est la phytase de la SEQ ID No. 1 ou la phytase de la SEQ ID No. 2.

De préférence, la deuxième phytase est une 3-phytase catalysant l'hydrolyse d'au moins cinq liaisons phosphate de l'acide phytique.

Préférentiellement, la deuxième phytase à une température optimum comprise entre 50°C et 60°C et un pH optimum compris entre pH 2 et pH 6.

Dans un mode de réalisation préféré, la deuxième phytase est une phytase d'*Aspergillus niger.*

Dans un autre mode de réalisation préféré, la deuxième phytase est la phytase de la SEQ ID No. 3.

Préférentiellement, les compositions selon l'invention consistent en un additif nutritionnel pour animaux ou en un aliment pour animaux.

L'invention a également pour objet, l'utilisation d'une composition selon l'invention pour la fabrication d'un additif nutritionnel pour animaux ou d'un aliment pour animaux.

L'invention a aussi pour objet l'utilisation d'une composition selon l'invention pour augmenter la disponibilité du phosphore phytique et améliorer la digestibilité des aliments pour animaux.

La présente invention concerne aussi un procédé d'hydrolyse de l'acide phytique (myo-inositol 1,2,3,4,5,6-hexakis phosphate) en monophosphates inorganiques, en myo-inositols de degré de phosphorylation inférieur et en myo-inositol libre comprenant les étapes suivantes :
- on dispose d'une première phytase présentant au moins 80% d'identité avec la phytase de la SEQ ID No. 1 ou présentant au moins 80 % d'identité avec la phytase de la SEQ ID No. 2 ;
- on dispose d'une deuxième phytase présentant au moins 80 % d'identité avec la phytase de la SEQ ID No. 3;
- on met simultanément en contact l'acide phytique avec la première phytase et la deuxième phytase.

De préférence, la première phytase est une 3-phytase catalysant l'hydrolyse des six liaisons phosphate de l'acide phytique.

Préférentiellement, la première phytase a une température optimum comprise entre 55°C et 80°C et un pH optimum compris entre pH 3.5 et pH 5.

Dans un mode de réalisation préféré, la première phytase est une phytase de la levure *Schwanniomyces castellii* ou une phytase de la levure *Debaryomyces castellii.*

Dans un mode de réalisation particulièrement avantageux, la première phytase est la phytase de la SEQ ID No. 1 ou la phytase de la SEQ ID No. 2.

De préférence, la deuxième phytase est une 3-phytase catalysant l'hydrolyse d'au moins cinq liaisons phosphate de l'acide phytique.

Préférentiellement, la deuxième phytase à une température optimum comprise entre 50°C et 60°C et un pH optimum compris entre pH 2 et pH 6.

Dans un mode de réalisation avantageux, la deuxième phytase est une phytase d'*Aspergillus niger.*

Dans un mode de réalisation particulièrement avantageux, la deuxième phytase est la phytase de la SEQ ID No. 3.

L'invention a aussi pour objet un kit ou ensemble pour l'alimentation des animaux comprenant :
- la phytase de *Schwanniomyces castellii* de la SEQ ID No. 1 ou la phytase de *Debaryomyces castellii* de la SEQ ID No. 2 ;
- la phytase d'*Aspergillus nigerde* la SEQ ID No. 3.

Les compositions selon l'invention associent une première phytase présentant au moins 80 % d'identité avec la phytase de la SEQ ID No. 1 ou présentant au moins 80 % d'identité avec la phytase de la SEQ ID No.2 ; et une deuxième phytase présentant au moins 80 % d'identité avec la phytase de la SEQ ID No. 3.

De façon surprenante, cette association spécifique de phytases permet d'obtenir un effet synergique sur l'hydrolyse de l'acide phytique. Les compositions selon la présente invention catalysent ainsi une hydrolyse rapide et totale de l'acide phytique dans une large gamme de pH.

On entend par phytase, les myo-inositol hexakis phosphate phosphohydrolases (EC 3.1.3.8 et 3.1.3.26). Ces enzymes catalysent l'hydrolyse du myo-inositol 1,2,3,4,5,6-hexakis phosphate (acide phytique, InsP₆) en monophosphates inorganiques et en myo-inositols phosphate de degré inférieur (InsP₅ à InsP₁) et en myo-inositol libre pour certaines phytases.

Les compositions selon l'invention comprennent une première phytase présentant au moins 80 % d'identité avec la phytase de la SEQ ID No. 1 ou présentant au moins 80 % d'identité avec la phytase de la SEQ ID No.2.

La phytase de la SEQ ID No. 1 est une phytase de la levure *Schwanniomyces castellii* alors que la phytase de la SEQ ID No. 2 est une phytase de la levure *Debaryomyces castellii.*

Ces phytases ont des propriétés catalytiques très proches et sont donc interchangeables dans les associations de phytases selon l'invention.

De préférence, la première phytase présente au moins 80 %, 90%, 95%, 98% et préférentiellement au moins 99% d'acides aminés identiques avec la phytase de la SEQ ID No. 1 ou présente au moins 80 %, 90%, 95%, 98% et préférentiellement au moins 99% d'acides aminés identiques avec la phytase de la SEQ ID No.2.

De préférence, la première phytase a les mêmes propriétés et notamment les mêmes propriétés catalytiques que les phytases de la SEQ ID No. 1 et de la SEQ ID No. 2. Préférentiellement, la première phytase est isolée d'autres souches de *Schwanniomyces castellii,* de *Debaryomyces castellii* ou d'autres levures. Alternativement, la première phytase peut être obtenue par des techniques de mutagenèse dirigée par exemple.

Par acides aminés identiques, on entend des acides aminés invariants entre deux séquences. La première phytase peut présenter une délétion, une addition ou une substitution d'au moins un acide aminé par rapport aux phytases de la SEQ ID No. 1 ou de la SEQ ID No. 2.

Les phytases de la SEQ ID No. 1 et de la SEQ ID No. 2 ont des propriétés catalytiques communes. Selon l'invention, la première phytase mise en oeuvre dans les compositions et les procédés selon l'invention présente un degré d'identité avec la phytase de la SEQ ID No.1 ou de la SEQ ID No. 2 et conserve ces propriétés catalytiques communes.

De préférence, la première phytase a une activité de 3-phytase. Préférentiellement, la première phytase catalyse l'hydrolyse des six liaisons phosphate de l'acide phytique.

Les 3-phytases (EC 3.1.3.8) hydrolysent en premier lieu le phosphate en position 3.

Une phytase capable d'hydrolyser toutes les liaisons phosphate de l'acide phytique (positions 1, 2, 3, 4, 5, 6) catalyse l'hydrolyse du myo-inositol 1,2,3,4,5,6-hexakis phosphate (acide phytique, InsP₆) en monophosphates inorganiques et en myo-inositol libre.

De nombreuses phytases n'hydrolysent que 5 liaisons phosphate de l'acide phytique soit 83% du phosphore potentiel. Ces enzymes hydrolysent l'acide phytique en myo-inositol monophosphate mais pas en myo-inositol libre.

Par "activité de la phytase", on entend l'activité enzymatique de la phytase. Cette activité s'exprime en Unités Internationales (U.I) par milligramme de protéine. Une U.I. d'activité enzymatique est la capacité catalytique à transformer une micromole de substrat par unité de temps, à un pH et à une température donnés.

De préférence, la première phytase a une température optimum comprise entre 55°C et 80°C et un pH optimum compris entre pH 3.5 et pH 5.

Dans un mode de réalisation préféré, la première phytase est une phytase de la levure *Schwanniomyces castellii* ou une phytase de la levure *Debaryomyces castellii_{.}*

Dans un autre mode de réalisation particulièrement avantageux, la première phytase est la phytase de la SEQ ID No. 1 ou la phytase de la SEQ ID No. 2.

Les compositions selon l'invention comprennent au moins une deuxième phytase présentant au moins 80% d'identité avec la phytase de la SEQ ID No. 3.

La phytase de la SEQ ID No. 3 est une phytase du champignon filamenteux *Aspergillus niger.*

De préférence, la deuxième phytase présente au moins 80 %, 90%, 95%, 98% et préférentiellement au moins 99% d'acides aminés identiques avec la phytase de la SEQ ID No. 3.

De préférence, la deuxième phytase conserve les propriétés et notamment les propriétés catalytiques de la phytase de la SEQ ID No. 3. Préférentiellement, cette phytase est isolée d'autres souches d'*Aspergillus niger* ou d'autres champignons filamenteux. Alternativement, cette phytase peut être obtenue par des techniques de mutagenèse dirigée par exemple.

Par "acides aminés identiques", on entend des acides aminés invariants entre deux séquences. La deuxième phytase peut présenter une délétion, une addition ou une substitution d'au moins un acide aminé par rapport à la phytase de la SEQ ID No. 3.

Selon l'invention, la deuxième phytase mise en oeuvre dans les compositions et les procédés selon l'invention présente donc un degré d'identité avec la phytase de la SEQ ID No. 3 et conserve les propriétés catalytiques de cette dernière.

De préférence, la deuxième phytase est une 3-phytase catalysant l'hydrolyse d'au moins cinq liaisons phosphate de l'acide phytique.

Préférentiellement, la deuxième phytase a une température optimum comprise entre 50°C et 60°C et un pH optimum compris entre pH 2 et pH 6. Encore plus préférentiellement, la deuxième phytase a un premier pH optimum entre pH 2.5 et 3.5 et un deuxième pH optimum entre pH 4.5 et 5.5.

Dans un mode de réalisation préféré, la deuxième phytase est une phytase d'*Aspergillus niger.*

Dans un mode de réalisation avantageux, la deuxième phytase est la phytase de la SEQ ID No. 3.

L'activité enzymatique d'une phytase varie notamment en fonction du pH. Les phytases ont ainsi des gammes (ou des plages) de pH dans lesquelles leur activité enzymatique est plus élevée ou optimale. Par gamme on entend une plage ou un intervalle de pH donné. Deux phytases ont des activités enzymatiques dans des gammes de pH complémentaires lorsque ces phytases ont une activité dans des gammes de pH différentes. De préférence, ces gammes se chevauchent ou se superposent partiellement. Typiquement, les phytases ont une activité enzymatique plus élevée à pH acide. Deux phytases ayant des gammes de pH complémentaires ont ainsi par exemple une activité enzymatique maximale entre pH 2 et pH 4 pour la première phytase et une activité enzymatique maximale entre pH 4 et pH 6 pour la deuxième phytase. Dans les compositions et les procédés de la présente invention, la première et la deuxième phytase ont de préférence des gammes de pH ou des pHs optimums complémentaires. Les compositions et les procédés selon l'invention permettent ainsi une hydrolyse de l'acide phytique dans une gamme de pH large.

Les méthodes de mesure et d'identification du degré d'identité entre polypeptides sont connues de l'homme du métier. On peut employer par exemple Vector NTi 9.1.0, programme d'alignement AlignX (Clustal W algorithm) (Invitrogen INFORMAX, http://www.invitrogen.com). De préférence, on utilise les paramètres par défaut.

Les phytases des compositions et procédés selon l'invention sont isolées ou purifiées de leur environnement naturel. Les phytases peuvent être préparés au moyen de différents procédés. Ces procédés sont notamment la purification à partir de sources naturelles telles que des cellules exprimant naturellement ces phytases, la production de phytases recombinantes par des cellules hôtes appropriées et leur purification ultérieure, la production par synthèse chimique ou, enfin, une combinaison de ces différentes approches. Ces différents procédés de production sont bien connus de l'homme du métier. Ainsi, les phytases mises en oeuvre dans les compositions et les procédés de la présente invention peuvent être isolées à partir de *Schwanniomyces castellii,* de *Debaryomyces castellii* ou d'*Aspergillus niger.* Dans un autre mode de réalisation, les phytases de la présente invention sont isolées à partir d'organismes hôtes recombinants.

Préférentiellement, les compositions selon l'invention consistent en un additif nutritionnel pour animaux ou en un aliment pour animaux.

L'invention a également pour objet l'utilisation d'une composition selon l'invention pour la fabrication d'un additif nutritionnel pour animaux ou d'un aliment pour animaux.

La présente invention concerne donc également des additifs alimentaires apportant une activité phytase. L'apport de ce type d'activité enzymatique permet d'améliorer la digestibilité de l'aliment et d'améliorer sa valeur nutritionnelle.

On entend par "additif nutritionnel" une substance ajoutée intentionnellement à un aliment, généralement en petites quantités, pour améliorer ses caractéristiques nutritionnelles ou sa digestibilité. Les additifs nutritionnels pour animaux peuvent par exemple contenir des vitamines, des sels minéraux, des acides aminés et des enzymes.

La présente invention concerne aussi les aliments pour animaux. Ces aliments se présentent habituellement sous la forme de farines ou de granulés dans lesquels sont incorporés les additifs selon l'invention. On entend par "aliment" tout ce qui peut servir à la nourriture des animaux. Pour l'élevage intensif des animaux, les aliments pour animaux comprennent habituellement une base nutritionnelle et des additifs nutritionnels. On entend par "base nutritionnelle", ce qui constitue l'essentiel de la ration alimentaire de l'animal, constitué à titre d'exemple par un mélange de céréales, de protéines et de matières grasses d'origine animale et/ou végétale.

Les bases nutritionnelles pour animaux sont adaptées à l'alimentation de ces animaux et sont bien connues de l'homme du métier. Habituellement, ces bases nutritionnelles comprennent par exemple du maïs, du blé, du pois et du soja. Ces bases nutritionnelles sont adaptées aux besoins des différentes espèces animales auxquelles elles sont destinées. Ces bases nutritionnelles peuvent déjà contenir des additifs nutritionnels comme des vitamines, des sels minéraux et des acides aminés.

Dans un mode de réalisation préféré, l'invention concerne des aliments pour animaux monogastriques et notamment pour les volailles et les porcs. Les volailles comprennent notamment les poules pondeuses, les poulets de chair, les dindes et les canards. Les porcs comprennent notamment les porcs croissance et finition ainsi que les porcelets.

L'invention a aussi pour objet l'utilisation d'une composition selon l'invention pour augmenter la disponibilité du phosphore phytique et améliorer la digestibilité des aliments pour animaux.

Les compositions et les procédés selon l'invention associent une première phytase capable d'hydrolyser toutes les liaisons phosphate de l'acide phytique, choisie parmi les phytases de *Schwanniomyces castellii* et les phytases de *Debaryomyces castellii* et une deuxième phytase capable d'hydrolyser au moins cinq liaisons phosphate de l'acide phytique et ayant une activité enzymatique dans une gamme de pH complémentaire à la gamme de pH de la première phytase.

### Figures

**Figure 1** : Comparaison des cinétiques d'hydrolyse de l'acide phytique à différents pH pour les 6 phytases seules ou associées deux à deux. Les résultats sont exprimés en % des phosphates théoriques libérés.
**Figure 2** : Pourcentage d'hydrolyse de l'acide phytique par les différentes phytases après 120 minutes de réaction. (A) phytases seules, (B) phytases associées. Les traits en pointillés correspondent à l'hydrolyse de 5 liaisons phosphate.
**Figure 3** : Comparaison des vitesses initiales d'hydrolyse de l'acide phytique (µmol.ml⁻¹.min⁻¹) pour 6 phytases à différents pH. (A) phytases seules, (B) phytases associées. Les vitesses sont calculées pendant les 10 premières minutes de la réaction.

### Exemples

### Matériels et methodes

### 1. Origines des phytases

■ *Aspergillus niger* Natuphos (AN): lot R2503 ou lot: 057NPHO2
   Wim van Hartingsveldt, Cora M. J. van Zeijl, G. Marian Harteveld, Robin J. Gouka, Marjon E. G. Suykerbuyk, Ruud G. M. Luiten, Peter A. van Paridon, Gerard C. M. Selten, Annemarie E. Veenstra, Robert F. M. van Gorcom and Cees A. M. J. J van den Hondel; Cloning, characterization and overexpression of the phytase-encoding gene (phyA) of Aspergillus niger. Gene, 127, (1) 87-94 (1993)
■ *Peniophora lycii* (PL): lot 172NHS02
   Lassen,S.F., Breinholt,J., Ostergaard,P.R., Brugger,R.,Bischoff,A., Wyss,M. and Fuglsang,C.C.; Expression, gene cloning, and characterization of five novel phytases from four basidiomycete fungi: Peniophora lycii, Agrocybe pediades, a Ceriporia sp., and Trametes pubescens. Appl. Environ. Microbiol. 67 (10), 4701-4707 (2001)
■ *Penicillium funiculosum* (PF): Godo A1346 lot 1301
   WO 03054199
■ Blé (B) : Sigma référencé P1259.
   WO 0183763
■ *Schwanniomyces castellii* (SC): produite dans *Candida boidinii* lot 10059 CN 25
   EP 0931837
■ *Debaryomyces castellii souche* CBS 2923 (DC).

### 2. Méthode enzymatique

L'activité phytasique est mesurée en suivant la libération de phosphate inorganique au cours du temps.

L'activité est mesurée en présence de 8 mM de phytate de sodium (Sigma) dissous dans un tampon acétate de sodium pH 5.5, 200 mM à 37°C (5 volumes). La réaction est déclenchée par l'addition de l'extrait enzymatique (1 volume). La réaction est stoppée par acidification du milieu par de l'acide trichloracétique 20% (1 volume de milieu réactionnel + 1 volume d'acide). La quantité de phosphate libéré est déterminée après différents temps d'incubation.

Une unité enzymatique (U) est définie comme la quantité d'enzyme qui libère une µmole de phosphate inorganique en une minute.

### 3. Condition d'hydrolyse des phytates à différents pH

L'hydrolyse est réalisée à 40°C dans 10 ml de tampon acétate 250 mM pour les pH 4 à 6.5 ou de tampon glycine 200 mM à pH 3, renfermant : 0,340 mM de phytate de sodium, 0,04 ou 0,08 U/mL de phytase. Des prélèvements sont réalisés à des temps différents pendant 120 minutes. La réaction est arrêtée par adition d'acide trichloracétique.

Les phosphates sont dosés directement sans dilution.

### 4. Dosage des phosphates

La quantité de phosphate libéré est révélée par colorimétrie. La solution de révélation, préparée extemporanément, contient du sulfate de fer (380 mM, 1 volume) et de l'heptamolybdate d'ammonium (12 mM, 4 volumes). La mesure de l'absorbance, à 700 nm, se fait après 30 minutes de révélation à température ambiante (1 volume de milieu réactionnel + 1 volume de solution de révélation), à l'aide d'un spectrophotomètre UV/visible (Beckman DU 530).

Une droite d'étalonnage est préalablement établie avec du potassium dihydrogénophosphate.

### Résultats

### 1. Phytases testées

Les phytases testées se distinguent selon différents critères : (1) caractéristiques biochimiques (pH optimum, stéréospécificité : 3, 4, ou 6 phytases), (2) nombre de liaisons phosphates hydrolysés.

Toutes ces phytases ont un pH optimum d'hydrolyse proche de 5, cependant les phytases de AN et PL ont un deuxième pH optimum à 2,5. Trois hydrolysent totalement les phosphates (SC, DC, Blé). La position hydrolysée en premier est soit la position 3 (AN, PL, DC), soit la position 6 (PL), soit la position 4 (Blé) **(Tableau 1)**.

**Tableau 1 : Comparaison des caractéristiques biochimiques des phytases**

| **Origine** | **Km (**µ**M)** | **pH optimum** | **Température optimum (°C)** | **Nombre de liaisons phosphate hydrolysé** | **Stéréospécificité** (ordre d'hydrolyse des liaisons phosphate) |
|---|---|---|---|---|---|
| *Aspergillus niger* ⁽¹⁾ | 40/27 | 2.5 - 5.5 | 55/58 | 5 | 3/4/5/6/1 |
| *Peniophora lycii* ⁽¹⁾ | 33 | 2.5 - 5.5 | 58 | 5 | 6/3 (pH5.5) 3/6 (pH3 et 5.5) |
| *Penicillium funiculosum* ⁽²⁾ | 550 | 4-5 | 50 | 5 ou 6 | 3/4 |
| Blé ⁽³⁾ | 22 | 5 - 7 | 55 | 6 | 4/3/5/6/1/2 |
| *castellii* ⁽⁴⁾ | *Schwanniomyces* 38 4.4 | | 77 | 6 ? | |
| *Debaryomyces castellii* | 236 | 4-4.5 | 60 | 6 | 3/4/5/6/1-2 |

| | | | | | |
|---|---|---|---|---|---|
| (1) Ullah, A. H. J and Sethumadhavan, K., (2003) PhyA gene product of Aspergillus ficuum and Peniophora lycii produces dissimilar phytases. Biochemical and Biophysical Research Communications 303 : 463-468 (2) WO 03054199 Filed Jun 13 2003 Bohlmann, R., Moussu, F., Nore, O., Pierrard, J., Saunier, D., Testeniere, O., New polynucleotide encoding fungal phytase, useful as feed additive to improve phosphate assimilation, also new strain of *Penicillium.* (3) Nakano, T., Joh, T., Narita, K., Hayakawa, T. (2000) The pathway of dephosphorylation of myo-Inositol Hexakisphosphate by Phytases from Wheat Bran of Tricicum aestivum L. cv Nourin#61. Biosci. Biotechnol. Biochem., 64:995-1003 (4) Segueilha, L., Lambrechts, C., Boze, H., Moulin, G. and Galzy, P. (1992) Purification and Properties of the Phytase from Schwanniomyces castellii. J. Ferment. Bioeng. 74(1): 7-11. | | | | | |

### 2. Comparaison de l'efficacité d'hydrolyse de l'acide phytique par 6 phytases à divers pH

L'étude est réalisée en présence de 0,340 mM de phytate. La quantité d'enzyme apportée correspond à 0,08 U/mL de phytase, mesurée dans des conditions standard à pH 5.5. Les valeurs de pH testées sont de 3, 4, 5.5, 6, 6.5. La réaction est suivie pendant 120 minutes. Des prélèvements sont effectués au cours du temps, la réaction est arrêtée par acidification, les phosphates sont dosés par colorimétrie.

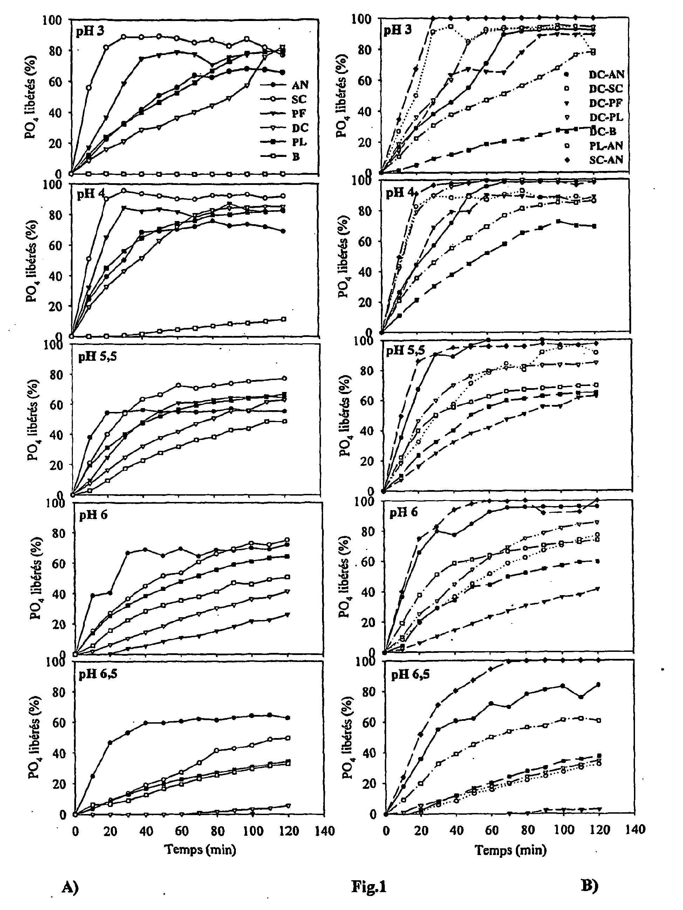

La comparaison des diverses phytases est effectuée en fonction de l'efficacité d'hydrolyse, c'est à dire du pourcentage de phosphate libéré par rapport au pourcentage théorique. Les résultats sont présentés dans la figure **1A****.**

D'après la littérature, 4 phytases (PF, SC, DC, B) sont capables d'hydrolyser totalement l'acide phytique en inositol et phosphate ; 2 phytases (AN et PL) n'hydrolysent que 5 liaisons soit 83% du phosphore potentiel.

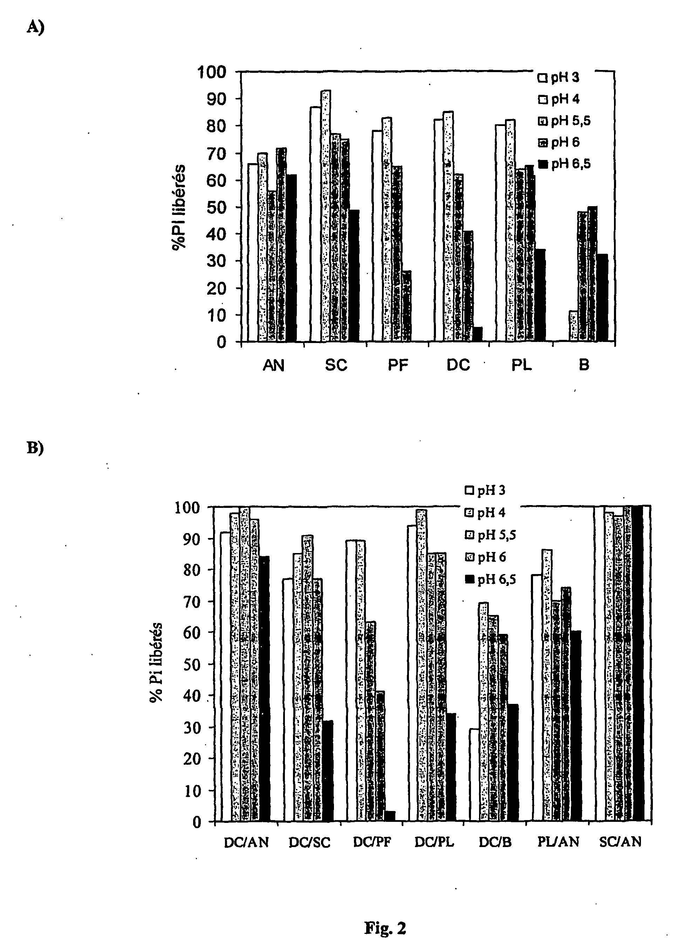

Dans les conditions testées à pH 3 et pH 4, 4 phytases (SC, PF, DC et PL) hydrolysent 80% au moins de l'acide phytique. La phytase de Blé est peu active aux pH 3 et 4. Pour les valeurs de pH supérieures à pH 5,5, aucune phytase n'hydrolyse plus de 70% de l'acide phytique. Les phytases PF et DC sont peu actives à pH 6,5. La phytase AN est la moins sensible aux variations de pH, cependant elle ne libère que 60 à 70 % des phosphates soit des valeurs 10 à 20 % inférieures à celles attendues (Fig 2A).

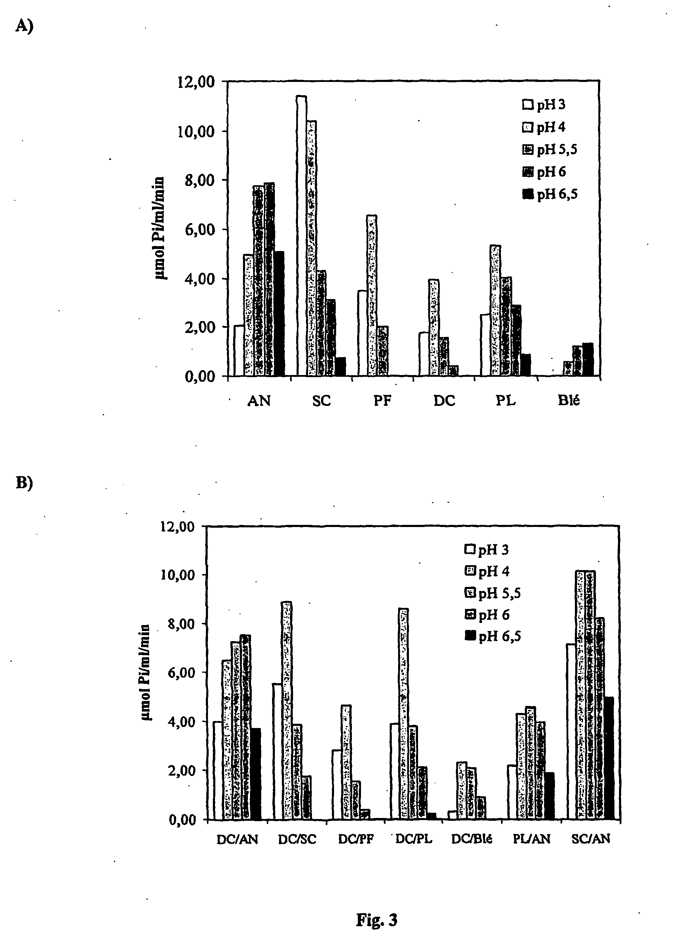

La comparaison des vitesses initiales de libération des phosphates montre une grande hétérogénéité entre les phytases (Fig 3A). La phytase la plus performante à pH acide est celle de *Schwaniomyces castellii* alors que celle de A. *niger* est la plus performante aux valeurs de pH supérieures à 5,5. La phytase de blé est la moins efficace quelles que soient les conditions testées.

### 3. Influence du pH sur la vitesse de libération de phosphate en présence de phytases associées.

Afin d'améliorer l'efficacité d'hydrolyse de l'acide phytique, plusieurs associations de deux phytases sont testées. Chaque phytase est apportée à 0,04 U/ml, soit une valeur totale de 0,08 U/ml dans le tube réactionnel. La phytase DC, hydrolysant 6 liaisons phosphate, est systématiquement testée en association avec les autres phytases, hydrolysant 5 ou 6 liaisons phosphate. Deux autres associations sont également utilisées pour comparer d'une part une autre association de phytases pouvant libérer 6 et 5 phosphates (SC/AN), d'autre part deux phytases, PUAN ayant des caractéristiques biochimiques très proches (Fig. 1B).

Une analyse globale permet de montrer que l'association de 2 phytases favorise la plupart du temps l'efficacité d'hydrolyse de l'acide phytique. Alors que dans les mêmes conditions, aucune phytase n'hydrolyse totalement l'acide phytique, pour 2 mélanges (DC/AN et SC/AN), 100% à 85% des liaisons phosphate sont hydrolysées quel que soit le pH. Pour l'association DC/PL on observe également un gain de 20 % environ aux pH acides. Les associations PL/AN, DC/PF et DC/SC apportent peu d'amélioration (Fig. 2B).

La comparaison des vitesses initiales d'hydrolyse, montre qu'il existe un fort effet synergique pour l'association SC/AN et pour l'association DC/AN par rapport aux phytases seules et par rapport aux autres associations de phytases. Les différences de vitesse d'hydrolyse liées au pH sont minimisées lors de l'association. Les autres associations sont moins efficaces, l'effet synergique n'est visible que pour certains pH, par exemple pour DC/PL aux pH 3 et 4 (Fig. 3).

L'effet synergique semble être dû en grande partie à la capacité des phytases à hydrolyser la totalité des phosphates. De plus, les meilleures associations concernent des phytases ayant des pH optimum d'activité complémentaires (2,5-4,5). Il faut cependant noter qu'il y a peu d'effet synergique pour les phytases les moins performantes en termes d'efficacité d'hydrolyse, telles que celle de Blé et de *Penicillium funiculosum.*

### 4. Influence des concentrations et de la proportion de phytases sur l'efficacité d'hydrolyse de l'acide phytique.

Nous avons comparé l'influence de la quantité de phytase (effets dose) et de la proportion du mélange de phytases sur l'hydrolyse de l'acide phytique. Trois associations sont utilisées DC/AN, DC/PL et AN/PL, et 2 concentrations (0,04 et 0,08 U/mL) en phytases sont testées (Tableaux 2 et 3).

**Tableau 2: Comparaison du pourcentage d'hydrolyse de l'acide phytique pour les différentes associations, testées à deux concentrations finales 0,04 et 0,08 U/ml. Les trois premières colonnes donnent les valeurs pour les trois phytases seules à la concentration 0,08 U/ml.**

| | **AN** (0,08U) | **DC** (0,08U) | **PL** (0,08U) | **AN/DC** (0,08U) | **AN/DC** (0,04U) | **PL/DC** 0,08U) | **PL/DC** (0,04U) | **AN/PL** (0,08U) | **AN/PL** (0,04U) |
|---|---|---|---|---|---|---|---|---|---|
| pH 3 | 66 | 82 | 80 | 92 | 67 *(73%)* | 94 | 70 *(74%)* | 79 | 56 *(72%)* |
| pH 4 | 69 | 85 | 83 | 95 | 96 *(101%)* | 100 | 93 *(94%)* | 86 | 76 *(89%)* |
| pH 5,5 | 55 | 63 | 67 | 92 | 81 *(87%)* | 85 | 71 *(84%)* | 70 | 66 *(94%)* |
| pH 6 | 72 | 42 | 64 | 94 | *78 (83%)* | 85 | *47 (56%)* | 74 | *67 (90%)* |
| pH 6,5 | 63 | 6 | 34 | 75 | 42 *(56%)* | 35 | 10 *(30%)* | 61 | 52 *(86%)* |

Les valeurs entre parenthèses représentent le rapport entre les valeurs obtenues avec 0,04 U/ml et celles en présence de 0,08 U/ml.

Les pourcentages d'hydrolyse lors de l'étude en mélange (concentration globale 0,08 U/ml) sont supérieurs à ceux déterminés avec les enzymes seules (0,08 U/ml) et proches de 100% pour les mélanges AN/DC et PUDC et cela dans une gamme de pH variant de 3 à 6. Le mélange AN/PL n'augmente pas de manière significative le pourcentage d'hydrolyse observé avec chaque phytase prise séparément. La réduction par un facteur 2 de la quantité globale apportée (0,04U/ml) n'induit pas une diminution par 2 des phosphates libérés. Dans certains cas, AN/DC et PUDC à pH 4 et pH 5,5, et AN/PL pH 4 à 6, les pourcentages d'hydrolyse sont équivalent à ceux observés en présence de 0,08U/ml (Tableau 3).

**Tableau 3 : Comparaison des vitesses initiales d'hydrolyse (µmol/ml/min) de l'acide phytique pour les différentes associations, testées à deux concentrations finales 0,04 et 0,08 U/ml. Les trois premières colonnes donnent les valeurs pour les trois phytases seules à la concentration 0,08 U/ml.**

| | **AN** (0,08 U) | **DC** (0,08 U) | **PL** (0,08 U) | **AN/DC** (0,08U) | **AN/DC** (0,04U) | **PL/DC** (0,08U) | **PL/DC** (0,04U) | **AN/PL** (0,08U) | **AN/PL** (0,04U) |
|---|---|---|---|---|---|---|---|---|---|
| pH 3 | 2,04 | 1,78 | 2,52 | 4,00 | 1,70 *(42%)* | 3,88 | 1,45 *(37%)* | 2,18 | 1,60 *(73%)* |
| pH 4 | 4,98 | 3,96 | 5,36 | 6,49 | 2,85 *(44%)* | 8,61 | 3,33 *(39%)* | 4,29 | 2,73 *(63%)* |
| pH 5,5 | 7,76 | 1,56 | 4,00 | 7,26 | 1,86 *(26%)* | 3,79 | 0,85 *(22%)* | 4,56 | 2,82 *(62%)* |
| pH 6 | 7,91 | 0,39 | 2,87 | 7,53 | 1,04 *(14%)* | 2,10 | 0,04 *(2%)* | 3,96 | 2,60 *(66%)* |
| pH 6,5 | 5,09 | 0,00 | 0,85 | 3,71 | 0,26 *(7%)* | 0,22 | 0,00 *(0%)* | 1,89 | 0,74 *(39%)* |

*Les valeurs entre parenthèses représentent le rapport entre les vitesses obtenues avec 0,04 U*/*ml et celles en présence de 0,08 U*/*ml.*

L'association de phytase permet donc de diminuer la quantité de phytase dans le milieu réactionnel par un facteur deux en maintenant une efficacité d'hydrolyse identique et cela dans une gamme de pH étendue. Il y a un net effet dose sur la libération de phosphate.

Par contre, les vitesses initiales d'hydrolyse de l'acide phytique sont au moins deux fois plus faible en présence de 0,04 U/mL de phytase qu'en présence de 0,08 U/mL (Tableau 3). Ces résultats confortent l'hypothèse que la synergie entre les phytases permet essentiellement l'hydrolyse totale des phosphates présents.

La proportion de chaque phytase dans le milieu réactionnel ne semble pas avoir beaucoup d'influence sur l'efficacité d'hydrolyse (Tableau 4). Dans tous les cas, une augmentation de la quantité de phosphate libéré est observée, avec un léger avantage pour une association à 50 % de chaque phytase, principalement pour les pH supérieur à 5,5.

**Tableau 4 : Comparaison du pourcentage d'hydrolyse de l'acide phytique en fonction de la proportion de chaque phytase dans le mélange. La quantité globale reste dans tous les cas de 0 ,08 U/ml.**

| Proportion de phytases | **AN** | **DC** | **AN/DC** (1/1) | **AN/DC** (1/2) | **AN/DC** (2/1) |
|---|---|---|---|---|---|
| pH 3 | 66 | 82 | 92 | 90 | 88 |
| pH 4 | 69 | 85 | 95 | 93 | 93 |
| pH 5,5 | 55 | 63 | 92 | 79 | 79 |
| pH 6 | 72 | 42 | 94 | 87 | 86 |
| pH 6,5 | 63 | 6 | 75 | 52 | 64 |

Dans nos conditions expérimentales, aucune phytase n'hydrolyse totalement l'acide phytique en inositol et phosphates, bien que 4 d'entre elles soient capables de libérer tous les phosphates selon la littérature. Quatre phytases, SC, PF, DC, PL hydrolysent 80% des liaisons phosphate aux valeurs de pH 3 et 4. Deux mélanges DC/AN et SC/AN hydrolysent de 80% à 100% des liaisons phosphate à tous les pH testés.

L'association de phytase de type, 3-phytase (AN, DC, PF), 4-phytase (B) ou 6-phytase (PL) entre elles n'apportent pas d'amélioration notable.

La complémentation et la synergie entre phytases semble dépendre de 2 critères propres à chaque phytase : (1) de leur profil d'activité en fonction du pH, (2) de leur capacité à hydrolyser la totalité ou non des phosphates.

L'utilisation de telles associations en nutrition animale pourrait permettre de diminuer les quantités de phytases utilisées d'au moins 20% et donc de diminuer les coûts. La teneur en phosphate d'origine phytique dans l'aliment serait également augmentée, améliorant la valeur alimentaire et permettrait ainsi de diminuer l'apport en phosphate inorganique. La pollution provoquée par les rejets de phosphate non disponible serait ainsi diminuée.

## Revendications

1. Composition associant au moins deux phytases pour l'hydrolyse de l'acide phytique (myo-inositol 1,2,3,4,5,6-hexakis phosphate) **caractérisée en ce qu'**elle comprend :
a) une première phytase présentant au moins 80% d'identité avec la phytase de la SEQ ID No. 1 ou présentant au moins 80 % d'identité avec la phytase de la SEQ ID No.2 qui est une 3-phytase catalysant l'hydrolyse des six liaisons phosphate de l'acide phytique;
b) une deuxième phytase présentant au moins 80 % d'identité avec la phytase de la SEQ ID No. 3 qui est une 3-phytase catalysant l'hydrolyse d'au moins cinq liaisons phosphate de l'acide phytique.

2. Composition selon la revendication précédente, **caractérisée en ce que** la première phytase a une température optimum comprise entre 55°C et 80°C et un pH optimum compris entre pH 3.5 et pH 5.

3. Composition selon l'une des revendications précédentes, **caractérisée en ce que** la première phytase est une phytase de la levure *Schwanniomyces castellii* ou une phytase de la levure *Debaryomyces castellii.*

4. Composition selon l'une des revendications précédentes, **caractérisée en ce que** la première phytase est la phytase de la SEQ ID No. 1 ou la phytase de la SEQ ID No. 2.

5. Composition selon l'une des revendications précédentes, **caractérisée en ce que** la deuxième phytase à une température optimum comprise entre 50°C et 60°C et un pH optimum compris entre pH 2 et pH 6.

6. Composition selon l'une des revendication précédentes, **caractérisée en ce que** la deuxième phytase est une phytase d'*Aspergillus niger.*

7. Composition selon l'une des revendications précédentes, **caractérisée en ce que** la deuxième phytase est la phytase de la SEQ ID No. 3.

8. Composition selon l'une des revendications précédentes, **caractérisée en ce qu'**elle consiste en un additif nutritionnel pour animaux ou en un aliment pour animaux.

9. Utilisation d'une composition selon l'une des revendications 1-7, pour la fabrication d'un additif nutritionnel pour animaux ou d'un aliment pour animaux.

10. Utilisation d'une composition selon l'une des revendications 1-8, pour augmenter la disponibilité du phosphore phytique et améliorer la digestibilité des aliments pour animaux.

11. Procédé d'hydrolyse de l'acide phytique (myo-inositol 1,2,3,4,5,6-hexakis phosphate) en monophosphates inorganiques, en myo-inositols de degré de phosphorylation inférieur et en myo-inositol libre **caractérisé en ce qu'**il comprend les étapes suivantes :
a) on dispose d'une première phytase présentant au moins 80% d'identité avec la phytase de la SEQ ID No. 1 ou présentant au moins 80 % d'identité avec la phytase de la SEQ ID No.2 qui est une 3-phytase catalysant l'hydrolyse des six liaisons phosphate de l'acide phytique;
b) on dispose d'une deuxième phytase présentant au moins 80 % d'identité avec la phytase de la SEQ ID No. 3 qui est une 3-phytase catalysant l'hydrolyse d'au moins cinq liaisons phosphate de l'acide phytique;
c) on met simultanément en contact l'acide phytique avec la première phytase et la deuxième phytase.

12. Procédé selon la revendication 11, **caractérisé en ce que** la première phytase a une température optimum comprise entre 55°C et 80°C et un pH optimum compris entre pH 3.5 et pH 5.

13. Procédé selon l'une des revendications 11 ou 12, **caractérisé en ce que** la première phytase est une phytase de la levure *Schwanniomyces castellii* ou une phytase de la levure *Debaryomyces castellii.*

14. Procédé selon l'une des revendications 11-13, **caractérisé en ce que** la première phytase est la phytase de la SEQ ID No. 1 ou la phytase de la SEQ ID No. 2.

15. Procédé selon l'une des revendications 11-14, **caractérisé en ce que** la deuxième phytase à une température optimum comprise entre 50°C et 60°C et un pH optimum compris entre pH 2 et pH 6.

16. Procédé selon l'une des revendications 11-15, **caractérisé en ce que** la deuxième phytase est une phytase d'*Aspergillus niger.*

17. Procédé selon l'une des revendications 11-16, **caractérisé en ce que** la deuxième phytase est la phytase de la SEQ ID No. 3.

18. Kit ou ensemble pour l'alimentation des animaux **caractérisé en ce qu'**il comprend :
a) la phytase de *Schwanniomyces castellii* de la SEQ ID No. 1 ou la phytase de *Debaryomyces castellii* de la SEQ ID No. 2 ;
b) la phytase d'*Aspergillus niger de* la SEQ ID No. 3.

## Claims

1. Composition combining at least two phytases for the hydrolysis of phytic acid (myo-inositol 1,2,3,4,5,6-hexakisphosphate), **characterized in that** it comprises:
a) a first phytase exhibiting at least 80% identity with the phytase of SEQ ID No. 1 or exhibiting at least 80% identity with the phytase of SEQ ID No. 2 which is a 3-phytase which catalyses the hydrolysis of the six phosphate bonds of phytic acid;
b) a second phytase exhibiting at least 80% identity with the phytase of SEQ ID No. 3 which is a 3-phytase which catalyses the hydrolysis of at least five phosphate bonds of phytic acid.

2. Composition according to the preceding claim, **characterized in that** the first phytase has an optimum temperature of between 55°C and 80°C and an optimum pH of between pH 3.5 and pH 5.

3. Composition according to either of the preceding claims, **characterized in that** the first phytase is a phytase of the yeast *Schwanniomyces castellii* or a phytase of the yeast *Debaryomyces castellii.*

4. Composition according to one of the preceding claims, **characterized in that** the first phytase is the phytase of SEQ ID No. 1 or the phytase of SEQ ID No. 2.

5. Composition according to one of the preceding claims, **characterized in that** the second phytase has an optimum temperature of between 50°C and 60°C and an optimum pH of between pH 2 and pH 6.

6. Composition according to one of the preceding claims, **characterized in that** the second phytase is an *Aspergillus niger* phytase.

7. Composition according to one of the preceding claims, **characterized in that** the second phytase is the phytase of SEQ ID No. 3.

8. Composition according to one of the preceding claims, **characterized in that** it consists of a nutritional additive for animals or of an animal feed.

9. Use of a composition according to one of Claims 1 to 7, for the manufacture of a nutritional additive for animals or of an animal feed.

10. Use of a composition according to one of Claims 1 to 8, for increasing the availability of phytic phosphorus and improving the digestibility of animal feeds.

11. Method of hydrolysis of phytic acid (myo-inositol 1,2,3,4,5,6-hexakisphosphate) to inorganic monophosphates, to myo-inositols with a lower degree of phosphorylation and to free myo-inositol, **characterized in that** it comprises the following steps:
a) a first phytase exhibiting at least 80% identity with the phytase of SEQ ID No. 1 or exhibiting at least 80% identity with the phytase of SEQ ID No. 2 which is a 3-phytase which catalyses the hydrolysis of the six phosphate bonds of phytic acid, is provided;
b) a second phytase exhibiting a least 80% identity with the phytase of SEQ ID No. 3 which is a 3-phytase which catalyses the hydrolysis of at least five phosphate bonds of phytic acid, is provided;
c) the phytic acid is simultaneously brought into contact with the first phytase and the second phytase.

12. Method according to Claim 11, **characterized in that** the first phytase has an optimum temperature of between 55°C and 80°C and an optimum pH of between pH 3.5 and pH 5.

13. Method according to either of Claims 11 and 12, **characterized in that** the first phytase is a phytase of the yeast *Schwanniomyces castellii* or a phytase of the yeast *Debaryomyces castellii.*

14. Method according to one of Claims 11 to 13, **characterized in that** the first phytase is the phytase of SEQ ID No. 1 or the phytase of SEQ ID No. 2.

15. Method according to one of Claims 11 to 14, **characterized in that** the second phytase has an optimum temperature of between 50°C and 60°C and an optimum pH of between pH 2 and pH 6.

16. Method according to one of Claims 11 to 15, **characterized in that** the second phytase is an *Aspergillus niger* phytase.

17. Method according to one of Claims 11 to 16, **characterized in that** the second phytase is the phytase of SEQ ID No. 3.

18. Kit or assembly for feeding animals, **characterized in that** it comprises:
a) the *Schwanniomyces castellii* phytase of SEQ ID No. 1 or the *Debaryomyces castellii* phytase of SEQ ID No. 2;
b) the *Aspergillus niger* phytase of SEQ ID No. 3.

## Patentansprüche

1. Zusammensetzung, bei der mindestens zwei Phytasen für die Hydrolyse von Phytinsäure (myo-Inositol-1,2,3,4,5,6-hexakisphosphat) kombiniert sind, **dadurch gekennzeichnet, daß** sie folgendes umfaßt:
a) eine erste Phytase mit mindestens 80% Identität zu der Phytase mit der SEQ ID NO. 1 oder mit mindestens 80% Identität zu der Phytase mit der SEQ ID NO. 2, bei der es sich um eine 3-Phytase, die die Hydrolyse der sechs Phosphatbindungen der Phytinsäure katalysiert, handelt;
b) eine zweite Phytase mit mindestens 80% Identität zu der Phytase mit der SEQ ID NO. 3, bei der es sich um eine 3-Phytase, die die Hydrolyse von mindestens fünf Phosphatbindungen der Phytinsäure katalysiert, handelt.

2. Zusammensetzung nach Anspruch 1, **dadurch gekennzeichnet, daß** die erste Phytase ein Temperaturoptimum zwischen 55°C und 80°C und ein pH-Optimum zwischen pH 3,5 und pH 5 aufweist.

3. Zusammensetzung nach einem der vorhergehenden Ansprüche, **dadurch gekennzeichnet, daß** es sich bei der ersten Phytase um eine Phytase aus der Hefe *Schwanniomyces castellii* oder um eine Phytase aus der Hefe *Debaryomyces castellii* handelt.

4. Zusammensetzung nach einem der vorhergehenden Ansprüche, **dadurch gekennzeichnet, daß** es sich bei der ersten Phytase um die Phytase mit der SEQ ID NO. 1 oder die Phytase mit der SEQ ID NO. 2 handelt.

5. Zusammensetzung nach einem der vorhergehenden Ansprüche, **dadurch gekennzeichnet, daß** die zweite Phytase ein Temperaturoptimum zwischen 50°C und 60°C und ein pH-Optimum zwischen pH 2 und pH 6 aufweist.

6. Zusammensetzung nach einem der vorhergehenden Ansprüche, **dadurch gekennzeichnet, daß** es sich bei der zweiten Phytase um eine Phytase von *Aspergillus niger* handelt.

7. Zusammensetzung nach einem der vorhergehenden Ansprüche, **dadurch gekennzeichnet, daß** es sich bei der zweiten Phytase um die Phytase mit der SEQ ID NO. 3 handelt.

8. Zusammensetzung nach einem der vorhergehenden Ansprüche, **dadurch gekennzeichnet, daß** sie aus einem Tierfuttermittelzusatz oder aus Tierfutter besteht.

9. Verwendung einer Zusammensetzung nach einem der Ansprüche 1-7 zur Herstellung eines Tierfuttermittelzusatzes oder von Tierfutter.

10. Verwendung einer Zusammensetzung nach einem der Ansprüche 1-8 zur Erhöhung der Verfügbarkeit von Phytinsäurephosphor und zur Verbesserung der Verdaulichkeit von Tierfutter.

11. Verfahren zur Hydrolyse von Phytinsäure (myo-Inositol-1,2,3,4,5,6-hexakisphosphat) zu anorganischen Monophosphaten, zu myo-Inositolen mit niedrigem Phosphorylierungsgrad und zu freiem myo-Inositol, **dadurch gekennzeichnet, daß** es die folgenden Schritte umfaßt:
a) man verfügt über eine erste Phytase mit mindestens 80% Identität zu der Phytase mit der SEQ ID NO. 1 oder mit mindestens 80% Identität zu der Phytase mit der SEQ ID NO. 2, bei der es sich um eine 3-Phytase, die die Hydrolyse der sechs Phosphatbindungen der Phytinsäure katalysiert, handelt;
b) man verfügt über eine zweite Phytase mit mindestens 80% Identität zu der Phytase mit der SEQ ID NO. 3, bei der es sich um eine 3-Phytase, die die Hydrolyse von mindestens fünf Phosphatbindungen der Phytinsäure katalysiert, handelt;
c) man bringt die Phytinsäure gleichzeitig mit der ersten Phytase und der zweiten Phytase in Kontakt.

12. Verfahren nach Anspruch 11, **dadurch gekennzeichnet, daß** die erste Phytase ein Temperaturoptimum zwischen 55°C und 80°C und ein pH-Optimum zwischen pH 3,5 und pH 5 aufweist.

13. Verfahren nach einem der Ansprüche 11 oder 12, **dadurch gekennzeichnet, daß** es sich bei der ersten Phytase um eine Phytase aus der Hefe *Schwanniomyces castellii* oder um eine Phytase aus der Hefe *Debaryomyces castellii* handelt.

14. Verfahren nach einem der Ansprüche 11-13, **dadurch gekennzeichnet, daß** es sich bei der ersten Phytase um die Phytase mit der SEQ ID NO. 1 oder die Phytase mit der SEQ ID NO. 2 handelt.

15. Verfahren nach einem der Ansprüche 11-14, **dadurch gekennzeichnet, daß** die zweite Phytase ein Temperaturoptimum zwischen 50°C und 60°C und ein pH-Optimum zwischen pH 2 und pH 6 aufweist.

16. Verfahren nach einem der Ansprüche 11-15, **dadurch gekennzeichnet, daß** es sich bei der zweiten Phytase um eine Phytase von *Aspergillus niger* handelt.

17. Verfahren nach einem der Ansprüche 11-16, **dadurch gekennzeichnet, daß** es sich bei der zweiten Phytase um die Phytase mit der SEQ ID NO. 3 handelt.

18. Kit oder Satz für die Tierfütterung, **dadurch gekennzeichnet, daß** es/er folgendes umfaßt:
a) die Phytase aus *Schwanniomyces castellii* mit der SEQ ID NO. 1 oder die Phytase aus *Debaryomyces castellii* mit der SEQ ID NO. 2;
b) die Phytase aus *Aspergillus niger* mit der SEQ ID NO. 3.


### SEQUENCE LISTING

<110> ADISSEO FRANCE SAS
<120> Association de phytases
<130> HL/SG/EP-50016
<150> FR05.07335
   <151> 2005-08-07
<160> 7
<170> PatentIn version 3.2
<210> 1
   <211> 461
   <212> PRT
   <213> Schwanniomyces castellii (Debaryomyces occidentallis)
<400> 1
   Met Val Ser Ile Ser Lys Leu Ile Asn Asn Gly Leu Leu Leu Ala Gly
   1               5                   10                  15
   Gln Ser Val Tyr Gln Asp Leu Ala Thr Pro Gln Gln Ser Ser Val Glu
               20                  25                  30
   Gln Tyr Asn Ile Ile Arg Phe Leu Gly Gly Ser Gly Pro Tyr Ile Gln
           35                  40                  45
   Arg Ser Gly Tyr Gly Ile Ser Thr Asp Ile Pro Asp Gln Cys Thr Ile
       50                  55                  60
   Lys Gln Val Gln Leu Met Ser Arg His Gly Glu Arg Tyr Pro Ser Lys
   65                  70                  75                  80
   Asn Ser Gly Lys Lys Leu Lys Thr Ile Tyr Gly Lys Leu Lys Ser Tyr
                   85                  90                  95
   Asn Gly Thr Phe Thr Gly Ser Leu Ala Phe Leu Asn Asp Tyr Glu Tyr
               100                 105                 110
   Phe Val Pro Asp Asp Ser Leu Tyr Glu Lys Glu Thr Ser Ala Leu Asn
           115                 120                 125
   Ser Gln Gly Leu Phe Ala Gly Thr Thr Asp Ala Leu Arg His Gly Ala
       130                 135                 140
   Ala Phe Arg Ala Lys Tyr Gly Ser Leu Tyr Lys Gln Asn Ser Thr Leu
   145 150 155 160
   Pro Val Phe Thr Ser Asn Ser Asn Arg Val Tyr Gln Thr Ser Glu Tyr
   165 170 175
   Phe Ala Arg Gly Phe Leu Gly Asp Glu Phe Ser Asp Ala Thr Val His
   180 185 190
   Phe Ala Ile Ile Asp Glu Asp Pro Lys Met Gly Val Asn Ser Leu Thr
   195 200 205
   Pro Arg Ala Ala Cys Asp Asn Tyr Asn Glu Asp Val Asn Asp Gly Ile
   210 215 220
   Val Asn Gln Tyr Ser Thr Asp Tyr Leu Asp Glu Ala Leu Lys Arg Phe
   225 230 235 240
   Gln Ser Ser Asn Pro Gly Leu Asn Leu Thr Ser Glu Asp Val Tyr Gln
   245 250 255
   Leu Phe Ala Tyr Cys Ala Tyr Glu Thr Asn Val Lys Gly Ala Ser Pro
   260 265 270
   Phe Cys Asp Leu Phe Thr Asn Glu Glu Tyr Ile Gln Tyr Ser Tyr Ser
   275 280 285
   Val Asp Leu Ser Asn Tyr Tyr Ser His Gly Ala Gly His Asn Leu Thr
   290 295 300
   Lys Thr Ile Gly Ser Thr Leu Leu Asn Ala Ser Leu Thr Leu Leu Lys
   305 310 315 320
   Asp Gly Thr Asn Asp Asn Lys Ile Trp Leu Ser Phe Ser His Asp Thr
   325 330 335
   Asp Leu Glu Ile Phe His Ser Ala Leu Gly Ile Val Glu Pro Ala Glu
   340 345 350
   Asp Leu Pro Val Asp Tyr Ile Pro Phe Pro Ser Pro Tyr Ile His Ser
   355 360 365
   Gln Ile Val Pro Gln Gly Ala Arg Ile Tyr Thr Glu Lys Tyr Ser Cys
   370 375 380
   Gly Asn Glu Thr Tyr Val Arg Tyr Ile Leu Asn Asp Ala Val Val Pro
   385 390 395 400
   Ile Pro Lys Cys Ser Ser Gly Pro Gly Phe Ser Cys Glu Leu Ser Lys
   405 410 415
   Phe Glu Glu Tyr Ile Asn Lys Arg Leu Arg Asp Val Asp Phe Val Glu
   420 425 430
   Gln Cys Asp Leu Lys Asp Ala Pro Thr Glu Val Thr Phe Tyr Trp Asp
   435 440 445
   Tyr Thr Ser Val Asn Tyr Ser Ala Ser Leu Ile Asn Gly
   450 455 460
<210> 2
   <211> 461
   <212> PRT
   <213> Debaryomyces castellii
<400> 2
   Met Val Ser Val Ser Lys Leu Ile Asn Asn Gly Leu Leu Leu Val Gly
   1               5                   10                  15
   Gln Gly Ala Tyr Gln Asp Leu Ala Ser Pro Gln Gln Ala Ser Val Glu
               20                  25                  30
   Gln Tyr Asn Ile Ile Arg Phe Leu Gly Gly Ala Ala Pro Tyr Ile Gln
           35                  40                  45
   Asn Lys Gly Phe Gly Ile Ser Thr Asp Ile Pro Asp Gln Cys Thr Leu
       50                  55                  60
   Glu Gln Val Gln Leu Phe Ser Arg His Gly Glu Arg Tyr Pro Ser Thr
   65                  70                  75                  80
   Gly Ser Gly Lys Lys Tyr Lys Ala Val Tyr Glu Lys Leu Met Ser Tyr
                   85                  90                  95
   Asn Gly Thr Phe Lys Gly Glu Leu Ala Phe Leu Asn Asp Asp Tyr Glu
               100                 105                 110
   Tyr Phe Val Pro Asp Ser Val Tyr Leu Glu Lys Glu Thr Ser Pro Lys
           115                 120                 125
   Asn Ser Asp Ser Ile Tyr Ala Gly Thr Thr Asp Ala Met Lys His Gly
   130 135 140
   Ile Ala Phe Arg Thr Lys Tyr Gly Glu Leu Phe Asp Thr Asn Asp Thr
   145 150 155 160
   Leu Pro Val Phe Thr Ser Asn Ser Gly Arg Val Tyr Gln Thr Ser Gln
   165 170 175
   Tyr Phe Ala Arg Gly Phe Met Gly Asp Asp Phe Ser Asn Asp Thr Val
   180 185 190
   Lys Thr Asn Ile Ile Ser Glu Asp Ala Asp Met Gly Ala Asn Ser Leu
   195 200 205
   Thr Pro Arg Asp Gly Cys Phe Asn Tyr Asn Glu Asn Ala Asn Thr Ala
   210 215 220
   Ile Val Asp Glu Tyr Thr Thr Glu Tyr Leu Thr Lys Ala Leu Asn Arg
   225 230 235 240
   Phe Lys Ala Ser Asn Pro Gly Leu Asn Ile Thr Glu Asp Asp Val Ser
   245 250 255
   Asn Leu Phe Gly Tyr Cys Ala Tyr Glu Leu Asn Val Lys Gly Ala Ser
   260 265 270
   Pro Met Cys Asp Ile Phe Thr Asn Glu Glu Phe Ile Gln Tyr Ser Tyr
   275 280 285
   Ser Val Asp Leu Asp Asp Tyr Tyr Ser Asn Ser Ala Gly Asn Asn Met
   290 295 300
   Thr Arg Val Ile Gly Ser Thr Leu Leu Asn Ala Ser Leu Glu Leu Leu
   305 310 315 320
   Asn His Asp Lys Asn Glu Asn Lys Ile Trp Leu Ser Phe Thr His Asp
   325 330 335
   Thr Asp Ile Glu Ile Phe His Ser Ala Ile Gly Ile Leu Ile Pro Asp
   340 345 350
   Glu Asp Leu Pro Val Asp Tyr Thr Pro Phe Pro Ser Pro Tyr Ser His
   355 360 365
   Val Gly Ile Thr Pro Gln Gly Ala Arg Thr Ile Ile Glu Lys Tyr Ala
       370                 375                 380
   Cys Gly Asn Glu Ser Tyr Val Arg Tyr Val Ile Asn Asp Ala Val Ile
   385                 390                 395                 400
   Pro Ile Lys Lys Cys Ser Ser Gly Pro Gly Phe Ser Cys Asn Leu Asn
                   405                 410                 415
   Asp Tyr Asn Asp Tyr Val Ala Glu Arg Val Ala Gly Thr Asn Tyr Val
               420                 425                 430
   Glu Gln Cys Gly Asn Asn Asn Ala Ser Ala Val Thr Phe Tyr Trp Asp
           435                 440                 445
   Tyr Glu Thr Thr Asn Tyr Thr Ala Ser Leu Ile Asn Ser
       450                 455                 460
<210> 3
   <211> 467
   <212> PRT
   <213> Aspergillus niger
<400> 3
   Met Gly Val Ser Ala Val Leu Leu Pro Leu Tyr Leu Leu Ser Gly Val
   1               5                   10                  15
   Thr Ser Gly Leu Ala Val Pro Ala Ser Arg Asn Gln Ser Ser Cys Asp
               20                  25                  30
   Thr Val Asp Gln Gly Tyr Gln Cys Phe Ser Glu Thr Ser His Leu Trp
           35                  40                  45
   Gly Gln Tyr Ala Pro Phe Phe Ser Leu Ala Asn Glu Ser Val Ile Ser
       50                  55                  60
   Pro Glu Val Pro Ala Gly Cys Arg Val Thr Phe Ala Gln Val Leu Ser
   65                  70                  75                  80
   Arg His Gly Ala Arg Tyr Pro Thr Asp Ser Lys Gly Lys Lys Tyr Ser
                   85                  90                  95
   Ala Leu Ile Glu Glu Ile Gln Gln Asn Ala Thr Thr Phe Asp Gly Lys
               100                 105                 110
   Tyr Ala Phe Leu Lys Thr Tyr Asn Tyr Ser Leu Gly Ala Asp Asp Leu
           115                 120                 125
   Thr Pro Phe Gly Glu Gln Glu Leu Val Asn Ser Gly Ile Lys Phe Tyr
       130                 135                 140
   Gln Arg Tyr Glu Ser Leu Thr Arg Asn Ile Val Pro Phe Ile Arg Ser
   145                 150                 155                 160
   Ser Gly Ser Ser Arg Val Ile Ala Ser Gly Lys Lys Phe Ile Glu Gly
                   165                 170                 175
   Phe Gln Ser Thr Lys Leu Lys Asp Pro Arg Ala Gln Pro Gly Gln Ser
               180                 185                 190
   Ser Pro Lys Ile Asp Val Val Ile Ser Glu Ala Ser Ser Ser Asn Asn
           195                 200                 205
   Thr Leu Asp Pro Gly Thr Cys Thr Val Phe Glu Asp Ser Glu Leu Ala
       210                 215                 220
   Asp Thr Val Glu Ala Asn Phe Thr Ala Thr Phe Val Pro Ser Ile Arg
   225                 230                 235                 240
   Gln Arg Leu Glu Asn Asp Leu Ser Gly Val Thr Leu Thr Asp Thr Glu
                   245                 250                 255
   Val Thr Tyr Leu Met Asp Met Cys Ser Phe Asp Thr Ile Ser Thr Ser
               260                 265                 270
   Thr Val Asp Thr Lys Leu Ser Pro Phe Cys Asp Leu Phe Thr His Asp
           275                 280                 285
   Glu Trp Ile Asn Tyr Asp Tyr Leu Gln Ser Leu Lys Lys Tyr Tyr Gly
       290                 295                 300
   His Gly Ala Gly Asn Pro Leu Gly Pro Thr Gln Gly Val Gly Tyr Ala
   305                 310                 315                 320
   Asn Glu Leu Ile Ala Arg Leu Thr His Ser Pro Val His Asp Asp Thr
                   325                 330                 335
   Ser Ser Asn His Thr Leu Asp Ser Ser Pro Ala Thr Phe Pro Leu Asn
               340                 345                 350
   Ser Thr Leu Tyr Ala Asp Phe Ser His Asp Asn Gly Ile Ile Ser Ile
   355 360 365
   Leu Phe Ala Leu Gly Leu Tyr Asn Gly Thr Lys Pro Leu Ser Thr Thr
   370 375 380
   Thr Val Glu Asn Ile Thr Gln Thr Asp Gly Phe Ser Ser Ala Trp Thr
   385 390 395 400
   Val Pro Phe Ala Ser Arg Leu Tyr Val Glu Met Met Gln Cys Gln Ala
   405 410 415
   Glu Gln Glu Pro Leu Val Arg Val Leu Val Asn Asp Arg Val Val Pro
   420 425 430
   Leu His Gly Cys Pro Val Asp Ala Leu Gly Arg Cys Thr Arg Asp Ser
   435 440 445
   Phe Val Arg Gly Leu Ser Phe Ala Arg Ser Gly Gly Asp Trp Ala Glu
   450 455 460
   Cys Phe Ala
   465
<210> 4
   <211> 540
   <212> PRT
   <213> Penicillium funiculosum
<400> 4
   Met Leu Lys Leu Tyr Val Ala Ala Cys Leu Val Val Ala Gly Val Ser
   1 5 10 15
   Ile Pro Thr Asp Pro Thr Val Thr Gln Val Pro Asp Tyr Phe Gln Thr
   20 25 30
   Ser Tyr Gly Pro Tyr Ala Gly Ala Thr Lys Ala Gly Gly Ala Pro Phe
   35 40 45
   Leu Ala Gln Thr Asn Pro Ile Tyr Ser Gln Pro Thr Tyr Val Ala Asn
   50 55 60
   Thr Pro Leu Val Thr Thr Leu Pro Ile Ser Gly Glu Pro His Asp Gly
   65 70 75 80
   Asn Ile Phe Gly Trp Met Gly Thr Leu Ser Pro Tyr Gln Pro Ser Pro
   85 90 95
   Asp Gly Phe Gly Val Asp Glu Tyr Pro Leu Pro Pro Gly Ala Asn Ile
   100 105 110
   Thr Gln Ile His Met Val His Arg His Gly Ser Arg Tyr Pro Thr Ser
   115 120 125
   Asn Ser Ala Ile Ser Asp Trp Ala Lys Lys Ile Met Gln Tyr Arg Ser
   130 135 140
   Asn Gly Thr Val Phe Ser Gly Glu Leu Glu Phe Leu Asn Ala Trp Asn
   145 150 155 160
   Tyr Gln Leu Gly Gln Ala Glu Leu Thr Ala Arg Gly Arg Gln Glu Leu
   165 170 175
   Phe Asp Ser Gly Ile Leu His Trp Phe Asn Tyr Gly Lys Leu Tyr Asp
   180 185 190
   Pro Ala Ser Lys Ile Ile Ala Arg Thr Thr Thr Met Val Arg Met Leu
   195 200 205
   Gln Ser Ala Glu Asn Phe Leu Asn Gly Phe Phe Gly Pro Asn Trp Thr
   210 215 220
   Asn Asn Ala Thr Leu Glu Val Ile Ile Glu Ser Thr Gly Phe Asn Asn
   225 230 235 240
   Ser Leu Ala Gly Asn Asp Met Cys Arg Asn Ala Lys Asn Thr Ser Gly
   245 250 255
   Gly Asp Ala Val Asn Glu Trp Thr Ala Leu Tyr Leu Gln Lys Ala Thr
   260 265 270
   Asn Arg Phe Arg Ser Glu Ile Ser Gly Ser Leu Asn Trp Thr Val Asp
   275 280 285
   Asp Thr Tyr Asn Ala Gln Ser Met Cys Pro Tyr Glu Thr Val Ala Leu
   290 295 300
   Gly Tyr Ser Pro Phe Cys Thr Leu Phe Ser Trp Glu Glu Trp Gln Gly
   305 310 315 320
   Phe Gln Tyr Val Asn Asp Leu Asn Leu Tyr Gly Asn Tyr Gly Met Gly
   325 330 335
   Ser Pro Val Gly Arg Ala Ile Gly Leu Gly Phe Val Glu Glu Leu Ile
   340 345 350
   Ala Arg Leu Gln Gly Gln Ile Pro Asn Pro Pro Glu Asp Ser Ile Gly
   355 360 365
   Phe Asn Gln Ser Leu Asp Asp Ser Ala Ala Thr Phe Pro Leu Asn Gln
   370 375 380
   Thr Ile Tyr Phe Asp Phe Ser His Asp Asn Glu Met Phe Ser Met Leu
   385 390 395 400
   Thr Ala Leu Gly Leu Thr Gln Phe Gly Asp Tyr Leu Ser Pro Thr Lys
   405 410 415
   Pro Ser Ala Asp Arg Ser Leu Ile Gly Ser His Ile Val Pro Phe Ser
   420 425 430
   Ala Thr Phe Val Phe Glu Ile Ile Lys Ala Pro Gly Leu Val Arg Glu
   435 440 445
   Asn Arg Ser Lys Tyr Cys Gly Glu Ser Val Tyr Glu Asn Thr Ser Glu
   450 455 460
   Glu Thr Thr Tyr Ile His Leu Val Ile Asn Gln Arg Thr Val Pro Leu
   465 470 475 480
   Gly Gln Ser Ile Ser Ala Cys Gly Gln Arg Asp Asp Gly Trp Cys Glu
   485 490 495
   Ile Ser Ala Phe Ile Gln Ala Gln Lys Glu Asn Ile Val Lys Ala Asn
   500 505 510
   Tyr Glu Glu Ser Cys Phe Gly Asn Trp Ser Ile Pro Ala Tyr Gly Glu
   515 520 525
   Ile Arg Asp Gly Ala Ile Pro Lys Asn Ala Thr Ser
   530 535 540
<210> 5
   <211> 439
   <212> PRT
   <213> Peniophora lycii
<400> 5
   Met Val Ser Ser Ala Phe Ala Pro Ser Ile Leu Leu Ser Leu Met Ser
   1               5                   10                  15
   Ser Leu Ala Leu Ser Thr Gln Phe Ser Phe Val Ala Ala Gln Leu Pro
               20                  25                  30
   Ile Pro Ala Gln Asn Thr Ser Asn Trp Gly Pro Tyr Asp Pro Phe Phe
           35                  40                  45
   Pro Val Glu Pro Tyr Ala Ala Pro Pro Glu Gly Cys Thr Val Thr Gln
       50                  55                  60
   Val Asn Leu Ile Gln Arg His Gly Ala Arg Trp Pro Thr Ser Gly Ala
   65                  70                  75                  80
   Arg Ser Arg Gln Val Ala Ala Val Ala Lys Ile Gln Met Ala Arg Pro
                   85                  90                  95
   Phe Thr Asp Pro Lys Tyr Glu Phe Leu Asn Asp Phe Val Tyr Lys Phe
               100                 105                 110
   Gly Val Ala Asp Leu Leu Pro Phe Gly Ala Asn Gln Ser His Gln Thr
           115                 120                 125
   Gly Thr Asp Met Tyr Thr Arg Tyr Ser Thr Leu Phe Glu Gly Gly Asp
       130                 135                 140
   Val Pro Phe Val Arg Ala Ala Gly Asp Gln Arg Val Val Asp Ser Ser
   145                 150                 155                 160
   Thr Asn Trp Thr Ala Gly Phe Gly Asp Ala Ser Gly Glu Thr Val Leu
                   165                 170                 175
   Pro Thr Leu Gln Val Val Leu Gln Glu Glu Gly Asn Cys Thr Leu Cys
               180                 185                 190
   Asn Asn Met Cys Pro Asn Glu Val Asp Gly Asp Glu Ser Thr Thr Trp
           195                 200                 205
   Leu Gly Val Phe Ala Pro Asn Ile Thr Ala Arg Leu Asn Ala Ala Ala
       210                 215                 220
   Pro Ser Ala Asn Leu Ser Asp Ser Asp Ala Leu Thr Leu Met Asp Met
   225                 230                 235                 240
   Cys Pro Phe Asp Thr Leu Ser Ser Gly Asn Ala Ser Pro Phe Cys Asp
                   245                 250                 255
   Leu Phe Thr Ala Glu Glu Tyr Val Ser Tyr Glu Tyr Tyr Tyr Asp Leu
               260                 265                 270
   Asp Lys Tyr Tyr Gly Thr Gly Pro Gly Asn Ala Leu Gly Pro Val Gln
           275                 280                 285
   Gly Val Gly Tyr Val Asn Glu Leu Leu Ala Arg Leu Thr Gly Gln Ala
       290                 295                 300
   Val Arg Asp Glu Thr Gln Thr Asn Arg Thr Leu Asp Ser Asp Pro Ala
   305                 310                 315                 320
   Thr Phe Pro Leu Asn Arg Thr Phe Tyr Ala Asp Phe Ser His Asp Asn
                   325                 330                 335
   Thr Met Val Pro Ile Phe Ala Ala Leu Gly Leu Phe Asn Ala Thr Ala
               340                 345                 350
   Leu Asp Pro Leu Lys Pro Asp Glu Asn Arg Leu Trp Val Asp Ser Lys
           355                 360                 365
   Leu Val Pro Phe Ser Gly His Met Thr Val Glu Lys Leu Ala Cys Ser
       370                 375                 380
   Gly Lys Glu Ala Val Arg Val Leu Val Asn Asp Ala Val Gln Pro Leu
   385                 390                 395                 400
   Glu Phe Cys Gly Gly Val Asp Gly Val Cys Glu Leu Ser Ala Phe Val
                   405                 410                 415
   Glu Ser Gln Thr Tyr Ala Arg Glu Asn Gly Gln Gly Asp Phe Ala Lys
               420                 425                 430
   Cys Gly Phe Val Pro Ser Glu
           435
<210> 6
   <211> 1623
   <212> DNA
   <213> Triticum aestivum
<220>
   <221> CDS
   <222> (1)..(1623)
<400> 6
   atg tgg atg tgg agg ggg tcg ctg ccg ctg ctt ctg ctc gcc gcg gcg       48
   Met Trp Met Trp Arg Gly Ser Leu Pro Leu Leu Leu Leu Ala Ala Ala
   1               5                   10                  15
   gtg gcg gcg gcg gct gag ccg gcg tcg acg ctg gag gga ccg tcg cgg       96
   Val Ala Ala Ala Ala Glu Pro Ala Ser Thr Leu Glu Gly Pro Ser Arg
               20                  25                  30
   ccg gtg acg gtg ccg ctg cgg gaa gac agg ggc cac gcg gtg gac ctg      144
   Pro Val Thr Val Pro Leu Arg Glu Asp Arg Gly His Ala Val Asp Leu
           35                  40                  45
   ccg gac acg gac ccc cgg gtg cag cgc cgg gtc aca ggc tgg gct ccc      192
   Pro Asp Thr Asp Pro Arg Val Gln Arg Arg Val Thr Gly Trp Ala Pro
       50                  55                  60
   gag cag atc gcc gtc gcg ctc tcc gcc gct ccc acc tcc gcc tgg gtc      240
   Glu Gln Ile Ala Val Ala Leu Ser Ala Ala Pro Thr Ser Ala Trp Val
   65                  70                  75                  80
   tcc tgg atc aca ggg gat ttc cag atg ggc ggc gcc gtc aag ccg ctg      288
   Ser Trp Ile Thr Gly Asp Phe Gln Met Gly Gly Ala Val Lys Pro Leu
                   85                  90                  95
   gac ccc ggc acg gtc ggc agc gtc gtg cgc tac ggc ctc gcc gcc gat      336
   Asp Pro Gly Thr Val Gly Ser Val Val Arg Tyr Gly Leu Ala Ala Asp
               100                 105                 110
   tct ttg gtc cgc gag gcc acc ggc gac gcg ctc gtg tac agc cag ctc      384
   Ser Leu Val Arg Glu Ala Thr Gly Asp Ala Leu Val Tyr Ser Gln Leu
           115                 120                 125
   tac ccc ttc gag ggc ctc cag aac tac acc tcc ggc atc atc cac cac      432
   Tyr Pro Phe Glu Gly Leu Gln Asn Tyr Thr Ser Gly Ile Ile His His
       130                 135                 140
   gtc cgc ctc caa ggg ctt gag cct ggg acg aag tac tac tac cag tgc      480
   Val Arg Leu Gln Gly Leu Glu Pro Gly Thr Lys Tyr Tyr Tyr Gln Cys
   145                 150                 155                 160
   ggc gac ccg gcc atc ccg ggg gcg atg agc gcc gtc cac gcg ttc cgg      528
   Gly Asp Pro Ala Ile Pro Gly Ala Met Ser Ala Val His Ala Phe Arg
                   165                 170                 175
   acg atg ccg gcg gtc ggg ccg cgg agc tac ccg ggg agg atc gcc gtg      576
   Thr Met Pro Ala Val Gly Pro Arg Ser Tyr Pro Gly Arg Ile Ala Val
               180                 185                 190
   gtg ggg gac ctc ggg ctc acg tac aac acc acc tcg acc gtg gac cac      624
   Val Gly Asp Leu Gly Leu Thr Tyr Asn Thr Thr Ser Thr Val Asp His
           195                 200                 205
   atg gcg agc aac cgg ccg gac ctg gtc ctc ctc gtc ggc gac gtg tgc      672
   Met Ala Ser Asn Arg Pro Asp Leu Val Leu Leu Val Gly Asp Val Cys
       210                 215                 220
   tac gcc aac atg tac ctc acc aac ggc acc gga gcg gac tgc tac tcg      720
   Tyr Ala Asn Met Tyr Leu Thr Asn Gly Thr Gly Ala Asp Cys Tyr Ser
   225                 230                 235                 240
   tgc gcg ttc ggc aag tcg acg ccc atc cac gag acg tac cag ccg cgc      768
   Cys Ala Phe Gly Lys Ser Thr Pro Ile His Glu Thr Tyr Gln Pro Arg
                   245                 250                 255
   tgg gac tac tgg gga agg tac atg gag gcg gtg acg tcg ggg acg ccg      816
   Trp Asp Tyr Trp Gly Arg Tyr Met Glu Ala Val Thr Ser Gly Thr Pro
               260                 265                 270
   atg atg gtg gtg gaa ggg aac cat gag ata gag gag cag atc ggg aac      864
   Met Met Val Val Glu Gly Asn His Glu Ile Glu Glu Gln Ile Gly Asn
           275                 280                 285
   aag acg ttc gcg gcc tac cgc tcc cgg ttc gcg ttc ccg tcg acg gag      912
   Lys Thr Phe Ala Ala Tyr Arg Ser Arg Phe Ala Phe Pro Ser Thr Glu
       290                 295                 300
   agc ggg tcc ttc tcc ccc ttc tac tac tcg ttc gac gcc ggc ggg atc      960
   Ser Gly Ser Phe Ser Pro Phe Tyr Tyr Ser Phe Asp Ala Gly Gly Ile
   305                 310                 315                 320
   cat ttc ctc atg ctc ggc gcc tac gcc gac tac ggc agg tca ggg gag     1008
   His Phe Leu Met Leu Gly Ala Tyr Ala Asp Tyr Gly Arg Ser Gly Glu
                   325                 330                 335
   cag tac aga tgg ctg gag aag gac ctg gcg aag gtg gac agg tcg gtg     1056
   Gln Tyr Arg Trp Leu Glu Lys Asp Leu Ala Lys Val Asp Arg Ser Val
               340                 345                 350
   acg ccg tgg ctg gtc gcc ggc tgg cac gcg cca tgg tac acc acc tac     1104
   Thr Pro Trp Leu Val Ala Gly Trp His Ala Pro Trp Tyr Thr Thr Tyr
           355                 360                 365
   aag gct cac tac agg gag gtg gag tgc atg aga gtg gcc atg gag gag     1152
   Lys Ala His Tyr Arg Glu Val Glu Cys Met Arg Val Ala Met Glu Glu
       370                 375                 380
   ctg ctc tac tcc cac ggc ctc gac atc gcc ttc acc ggc cat gtg cac     1200
   Leu Leu Tyr Ser His Gly Leu Asp Ile Ala Phe Thr Gly His Val His
   385                 390                 395                 400
   gcg tat gag cgc tcc aac cgg gtg ttc aac tac acg ctg gac ccg tgc     1248
   Ala Tyr Glu Arg Ser Asn Arg Val Phe Asn Tyr Thr Leu Asp Pro Cys
                   405                 410                 415
   ggc gcc gtg cac atc tcg gtg ggc gac ggc ggg aac cgc gag aag atg     1296
   Gly Ala Val His Ile Ser Val Gly Asp Gly Gly Asn Arg Glu Lys Met
               420                 425                 430
   gcc acc acc cac gcc gac gag cca ggg cac tgc ccg gac ccg cgg ccc      1344
   Ala Thr Thr His Ala Asp Glu Pro Gly His Cys Pro Asp Pro Arg Pro
           435                 440                 445
   aag ccc aac gcc ttc atc ggc ggc ttc tgc gcc ttt aac ttc acg tcc      1392
   Lys Pro Asn Ala Phe Ile Gly Gly Phe Cys Ala Phe Asn Phe Thr Ser
       450                 455                 460
   ggc ccg gcc gcc ggc agg ttc tgc tgg gac cgg cag ccg gac tac agc      1440
   Gly Pro Ala Ala Gly Arg Phe Cys Trp Asp Arg Gln Pro Asp Tyr Ser
   465                 470                 475                 480
   gcc tac cgg gag agc agc ttc ggc cac ggc atc ctc gag gtg aag aac      1488
   Ala Tyr Arg Glu Ser Ser Phe Gly His Gly Ile Leu Glu Val Lys Asn
                   485                 490                 495
   gag acg cac gct ctg tgg aga tgg cac agg aac cag gac atg tac ggg      1536
   Glu Thr His Ala Leu Trp Arg Trp His Arg Asn Gln Asp Met Tyr Gly
               500                 505                 510
   agc gcc gga gat gag att tac att gtc cgg gag ccg cac agg tgc ttg      1584
   Ser Ala Gly Asp Glu Ile Tyr Ile Val Arg Glu Pro His Arg Cys Leu
           515                 520                 525
   cac aaa cac aac tgg acc agg ccc gca cac ggt ccg taa                  1623
   His Lys His Asn Trp Thr Arg Pro Ala His Gly Pro
       530                 535                 540
<210> 7
   <211> 540
   <212> PRT
   <213> Triticum aestivum
<400> 7
   Met Trp Met Trp Arg Gly Ser Leu Pro Leu Leu Leu Leu Ala Ala Ala
   1               5                   10                  15
   Val Ala Ala Ala Ala Glu Pro Ala Ser Thr Leu Glu Gly Pro Ser Arg
               20                  25                  30
   Pro Val Thr Val Pro Leu Arg Glu Asp Arg Gly His Ala Val Asp Leu
           35                  40                  45
   Pro Asp Thr Asp Pro Arg Val Gln Arg Arg Val Thr Gly Trp Ala Pro
       50                  55                  60
   Glu Gln Ile Ala Val Ala Leu Ser Ala Ala Pro Thr Ser Ala Trp Val
   65                  70                  75                  80
   Ser Trp Ile Thr Gly Asp Phe Gln Met Gly Gly Ala Val Lys Pro Leu
   85 90 95
   Asp Pro Gly Thr Val Gly Ser Val Val Arg Tyr Gly Leu Ala Ala Asp
   100 105 110
   Ser Leu Val Arg Glu Ala Thr Gly Asp Ala Leu Val Tyr Ser Gln Leu
   115 120 125
   Tyr Pro Phe Glu Gly Leu Gln Asn Tyr Thr Ser Gly Ile Ile His His
   130 135 140
   Val Arg Leu Gln Gly Leu Glu Pro Gly Thr Lys Tyr Tyr Tyr Gln Cys
   145 150 155 160
   Gly Asp Pro Ala Ile Pro Gly Ala Met Ser Ala Val His Ala Phe Arg
   165 170 175
   Thr Met Pro Ala Val Gly Pro Arg Ser Tyr Pro Gly Arg Ile Ala Val
   180 185 190
   Val Gly Asp Leu Gly Leu Thr Tyr Asn Thr Thr Ser Thr Val Asp His
   195 200 205
   Met Ala Ser Asn Arg Pro Asp Leu Val Leu Leu Val Gly Asp Val Cys
   210 215 220
   Tyr Ala Asn Met Tyr Leu Thr Asn Gly Thr Gly Ala Asp Cys Tyr Ser
   225 230 235 240
   Cys Ala Phe Gly Lys Ser Thr Pro Ile His Glu Thr Tyr Gln Pro Arg
   245 250 255
   Trp Asp Tyr Trp Gly Arg Tyr Met Glu Ala Val Thr Ser Gly Thr Pro
   260 265 270
   Met Met Val Val Glu Gly Asn His Glu Ile Glu Glu Gln Ile Gly Asn
   275 280 285
   Lys Thr Phe Ala Ala Tyr Arg Ser Arg Phe Ala Phe Pro Ser Thr Glu
   290 295 300
   Ser Gly Ser Phe Ser Pro Phe Tyr Tyr Ser Phe Asp Ala Gly Gly Ile
   305 310 315 320
   His Phe Leu Met Leu Gly Ala Tyr Ala Asp Tyr Gly Arg Ser Gly Glu
                   325                 330                 335
   Gln Tyr Arg Trp Leu Glu Lys Asp Leu Ala Lys Val Asp Arg Ser Val
               340                 345                 350
   Thr Pro Trp Leu Val Ala Gly Trp His Ala Pro Trp Tyr Thr Thr Tyr
           355                 360                 365
   Lys Ala His Tyr Arg Glu Val Glu Cys Met Arg Val Ala Met Glu Glu
       370                 375                 380
   Leu Leu Tyr Ser His Gly Leu Asp Ile Ala Phe Thr Gly His Val His
   385                 390                 395                 400
   Ala Tyr Glu Arg Ser Asn Arg Val Phe Asn Tyr Thr Leu Asp Pro Cys
                   405                 410                 415
   Gly Ala Val His Ile Ser Val Gly Asp Gly Gly Asn Arg Glu Lys Met
               420                 425                 430
   Ala Thr Thr His Ala Asp Glu Pro Gly His Cys Pro Asp Pro Arg Pro
           435                 440                 445
   Lys Pro Asn Ala Phe Ile Gly Gly Phe Cys Ala Phe Asn Phe Thr Ser
       450                 455                 460
   Gly Pro Ala Ala Gly Arg Phe Cys Trp Asp Arg Gln Pro Asp Tyr Ser
   465                 470                 475                 480
   Ala Tyr Arg Glu Ser Ser Phe Gly His Gly Ile Leu Glu Val Lys Asn
                   485                 490                 495
   Glu Thr His Ala Leu Trp Arg Trp His Arg Asn Gln Asp Met Tyr Gly
               500                 505                 510
   Ser Ala Gly Asp Glu Ile Tyr Ile Val Arg Glu Pro His Arg Cys Leu
           515                 520                 525
   His Lys His Asn Trp Thr Arg Pro Ala His Gly Pro
       530                 535                 540